# EUROPEAN PATENT APPLICATION

(11) **EP 3 967 296 A1**
(43) Date of publication of application: **16.03.2022**
(21) Application number: 20802958.7
(22) Date of filing: 17.04.2020
(51) Int. Cl.: A61K 8/81, A61K 8/34, A61K 9/00, A61K 47/10, A61K 47/32, A61Q 19/00

(54) **VISCOUS COMPOSITION**

(30) Priority: 09.05.2019 JP 2019089008
(71) Applicant: Sumitomo Seika Chemicals Co., Ltd., Kako-gun, Hyogo 675-0145 (JP)
(72) Inventor: UEZUMI, Chiaki, Himeji-shi, Hyogo 672-8076 (JP)
(74) Representative: Vossius & Partner Patentanwälte Rechtsanwälte mbB
(86) International application number: PCT/JP2020/016936
(87) International publication number: WO 2020/226040

(57) **Abstract**

Provided is a viscous composition that does not lose its ease of handling even after long-term storage and is easily returned to a uniform composition even if layer separation occurs, despite containing a high concentration of a partially neutralized product of a polymer containing ethylenically unsaturated carboxylic acid units.

More specifically, provided is a viscous composition comprising (A) 10 to 40 mass% of a partially neutralized product of a polymer containing ethylenically unsaturated carboxylic acid units, (B) 0 to 50 mass% of glycerol, (C) 5 to 65 mass% of a dihydric alcohol, and (D) 0 to 20 mass% of water, the total of the components (B) and (C) being 40 mass% or more, the mass ratio of the total of the components (B) and (C) to the component (A), ((B)+(C))/(A), being 1.5 or more.

## Description

### Technical Field

The present disclosure relates to a viscous composition and the like. The contents of all of the documents described herein are incorporated herein by reference.

### Background Art

Viscous compositions (e.g., gelled compositions) are widely used in, for example, pharmaceuticals for external use and cosmetics. Viscous compositions are often prepared using thickening agents. As such thickening agents, for example, hydrophilic thickening agents are widely used. Specific examples thereof include natural thickening agents, such as xanthan gum and guar gum; semisynthetic thickening agents, such as hydroxyethyl cellulose and carboxy methylcellulose; and synthetic thickening agents, such as carboxyvinyl polymers and polyethylene oxide. Of these thickening agents, partially neutralized products of polymers containing ethylenically unsaturated carboxylic acid units are heavily used due to their low price, strong thickening effect, and ability to form a gel in small amounts.

### Citation List

### Patent Literature

PTL 1: JP2010-037272A
PTL 2: JP2010-265180A
PTL 3: JP2003-300826A
PTL 4: WO2017/022702

### Summary of Invention

### Technical Problem

As mentioned above, partially neutralized products of polymers containing ethylenically unsaturated carboxylic acid units have an excellent thickening effect, and a viscous composition can be obtained by dissolving such a partially neutralized product in water or the like. However, since the thickening effect is dramatically increased by increasing the amount of a partially neutralized product of a polymer containing ethylenically unsaturated carboxylic acid units, if a large amount of a partially neutralized product of a polymer containing ethylenically unsaturated carboxylic acid units is incorporated into water, the viscosity becomes too high, the dispersion in water becomes non-uniform, or layer separation occurs, resulting in a significant decrease in ease of handling. For this reason, a viscous composition containing a partially neutralized product of a polymer containing ethylenically unsaturated carboxylic acid units at a concentration of about 0.5 to 2 mass%, or at most about 4 to 5 mass%, is usually prepared before use, and then used for the production of products (e.g., cosmetics).

On the other hand, the higher the concentration of a viscous composition in which a partially neutralized product of a polymer containing ethylenically unsaturated carboxylic acid units is dissolved in water, the higher the efficiency in the production of products such as cosmetics. It is thus advantageous if a viscous composition can be prepared that does not lose its ease of handling even after long-term storage despite containing a high concentration of a partially neutralized product of a polymer containing ethylenically unsaturated carboxylic acid units.

### Solution to Problem

The present inventors found that a viscous composition in which a partially neutralized product of a polymer containing ethylenically unsaturated carboxylic acid units, and specific polyhydric alcohols (specifically, glycerol and a dihydric alcohol) are incorporated into a specific amount of water does not lose its ease of handling even after long-term storage and is easily returned to a uniform composition even if layer separation occurs, despite containing a high concentration of the partially neutralized product of a polymer containing ethylenically unsaturated carboxylic acid units. The inventors further made improvements.

The present disclosure includes, for example, the subject matter described in the following items.
Item 1. A viscous composition comprising:
   (A) 10 to 40 mass% of a partially neutralized product of a polymer containing ethylenically unsaturated carboxylic acid units;
   (B) 0 to 50 mass% of glycerol;
   (C) 5 to 65 mass% of a dihydric alcohol; and
   (D) 0 to 20 mass% of water,
      the total of the components (B) and (C) being 40 mass% or more,
      the mass ratio of the total of the components (B) and (C) to the component (A), ((B)+(C))/(A), being 1.5 or more.
Item 2. The viscous composition according to Item 1, wherein the mass ratio of the component (C) to the component (B), (C)/(B), is 0.5 to 5.
Item 3. The viscous composition according to Item 1 or 2, wherein the partially neutralized product of a polymer containing ethylenically unsaturated carboxylic acid units is crosslinked or is not crosslinked by a water-soluble crosslinking agent.
Item 4. The viscous composition according to Item 1 or 2, wherein the partially neutralized product of a polymer containing ethylenically unsaturated carboxylic acid units is crosslinked by a water-soluble crosslinking agent, and the water-soluble crosslinking agent is at least one member selected from the group consisting of ethylene glycol diglycidyl ether, pentaerythritol allyl ether, and sucrose allyl ether.
Item 5. The viscous composition according to any one of Items 1 to 4, wherein the dihydric alcohol (C) is present in an amount of 20 to 65 mass%.
Item 6. The viscous composition according to any one of Items 1 to 5, wherein the mass ratio of the total of the components (B) and (C) to the component (A), ((B)+(C))/(A), is 1.5 to 3.
Item 7. The viscous composition according to any one of Items 1 to 6, wherein the partially neutralized product of a polymer containing ethylenically unsaturated carboxylic acid units is a partially neutralized product of a (meth)acrylic acid polymer.
Item 8. The viscous composition according to any one of Items 1 to 7, wherein the partially neutralized product of a polymer containing ethylenically unsaturated carboxylic acid units is a neutralized product in which 30 to 80 mol% of all the ethylenically unsaturated carboxylic acid units contained in the polymer are neutralized.
Item 9. The viscous composition according to any one of Items 1 to 8, wherein the dihydric alcohol (C) is at least one member selected from the group consisting of propylene glycol, 1,3-butylene glycol, and pentylene glycol.
Item 10. The viscous composition according to Item 1, wherein the partially neutralized product of a polymer containing ethylenically unsaturated carboxylic acid units (A) is (i) a partially neutralized product of a (meth)acrylic acid polymer that is not crosslinked by a water-soluble crosslinking agent or (ii) a partially neutralized product of a (meth)acrylic acid polymer that is crosslinked by at least one water-soluble crosslinking agent selected from the group consisting of ethylene glycol diglycidyl ether, pentaerythritol allyl ether, and sucrose allyl ether;
   the partially neutralized product is a neutralized product in which 30 to 80 mol% of (meth)acrylic acid units contained in the (meth)acrylic acid polymer are neutralized;
   the dihydric alcohol (C) is at least one dihydric alcohol selected from the group consisting of propylene glycol, 1,3-butylene glycol, and pentylene glycol, and is present in an amount of 20 to 65 mass%;
   the mass ratio of the component (C) to the component (B), (C)/(B), is 0.5 to 5; and
   the mass ratio of the total of the components (B) and (C) to the component (A), ((B)+(C))/(A), is 1.5 to 3.

### Advantageous Effects of Invention

Provided is a viscous composition that does not lose its ease of handling even after long-term storage and is easily returned to a uniform composition even if layer separation occurs, despite containing a high concentration of a partially neutralized product of a polymer containing ethylenically unsaturated carboxylic acid units.

### Description of Embodiments

Embodiments included in the present disclosure are described in more detail below. The present disclosure preferably includes a viscous composition comprising specific components in specific amounts, a method for producing the viscous composition, and the like, but is not limited thereto. The present disclosure includes everything disclosed in the present specification and recognizable to those skilled in the art.

The viscous composition included in the present disclosure comprises (A) 10 to 40 mass% of a partially neutralized product of a polymer containing ethylenically unsaturated carboxylic acid units, (B) 0 to 50 mass% of glycerol, (C) 5 to 65 mass% of a dihydric alcohol, and (D) 0 to 20 mass% of water; further, the total of the components (B) and (C) is 40 mass% or more, and the mass ratio of the total of the components (B) and (C) to the component (A), ((B)+(C))/(A), is 1.5 or more. The viscous composition may be referred to as "the viscous composition of the present disclosure."

As described above, the partially neutralized product of a polymer containing ethylenically unsaturated carboxylic acid units (which may be simply referred to as "the component (A)") is present in an amount of 10 to 40 mass%. The upper or lower limit of the content range may be, for example, 11, 12, 13, 14, 15, 16, 17, 18, 19, 20, 21, 22, 23, 24, 25, 26, 27, 28, 29, 30, 31, 32, 33, 34, 35, 36, 37, 38, or 39 mass%. The content of the partially neutralized product of a polymer containing ethylenically unsaturated carboxylic acid units is preferably, for example, 15 to 37 mass%, 20 to 35 mass%, or 25 to 35 mass%.

The component (A) contains repeating structural units derived from an ethylenically unsaturated carboxylic acid monomer (ethylenically unsaturated carboxylic acid units). Some of the carboxyl groups in these repeating structural units are neutralized to form salts (e.g., metal salts or ammonium salts) (i.e., a partially neutralized product).

Examples of the ethylenically unsaturated carboxylic acid as a monomer include (meth)acrylic acid; 2-(meth)acrylamide-2-methylpropanesulfonic acid; nonionic monomers, such as (meth)acrylamide, N,N-dimethylacrylamide, 2-hydroxyethyl (meth)acrylate, and N-methylol (meth)acrylamide; amino-containing unsaturated monomers, such as diethylaminoethyl (meth)acrylate and diethylamino propyl(meth)acrylate; and the like. Of these, (meth)acrylic acid (i.e., methacrylic acid and/or acrylic acid) is preferable.

In the component (A), it is preferred that 30 to 80 mol% of all the repeating structural units derived from the ethylenically unsaturated carboxylic acid monomer are neutralized. The upper or lower limit of the range of the degree of neutralization (mol%) may be, for example, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, or 79 mol%. The range of the degree of neutralization (mol%) is more preferably, for example, 35 to 75 mol% or 40 to 75 mol%.

The partially neutralized product of a polymer containing ethylenically unsaturated carboxylic acid units may be crosslinked by a water-soluble crosslinking agent. Examples of water-soluble crosslinking agents include compounds having two or more reactive functional groups and/or polymerizable unsaturated groups, and the like. Specific examples include a glycidyl group, an isocyanate group, and the like. Examples of compounds having two or more glycidyl groups include ethylene glycol diglycidyl ether, polyethylene glycol diglycidyl ether, and the like. Examples of compounds having two or more polymerizable unsaturated groups include N,N'-methylenebis acrylamide, ethylene glycol dimethacrylate, polyethylene glycol dimethacrylate, pentaerythritol allyl ether, sucrose allyl ether, and the like. Preferable examples of the pentaerythritol allyl ether include pentaerythritol triallyl ether and pentaerythritol tetraallyl ether. The water-soluble crosslinking agents may be used singly, or in a combination of two or more.

When sucrose allyl ether is used, the degree of etherification is not particularly limited, and may be, for example, 2.0 to 3.5. The degree of etherification within this range facilitates the crosslinking reaction due to the presence of sufficient functional groups involved in the crosslinking reaction (i.e., allyl groups). The degree of etherification within this range also makes the solubility in water unlikely to decrease, thereby facilitating the crosslinking reaction of the sucrose allyl ether with the ethylenically unsaturated carboxylic acid monomer in an aqueous phase. The degree of etherification is more preferably 2.0 to 3.0.

As used here, the degree of etherification is defined as the average of molar ratios of allyl ether groups to sucrose. Specifically, hydroxyl groups remaining in sucrose allyl ether are reacted with acetic anhydride in pyridine, and the degree of etherification is calculated from the amount of acetic anhydride consumed during this reaction.

Sucrose allyl ether can be produced by a known method. For example, sucrose allyl ether can be produced by a method in which sodium hydroxide is added to a sucrose aqueous solution to convert sucrose into alkaline sucrose, and allyl bromide is added thereto to perform etherification. Sucrose allyl ether can be efficiently obtained by adjusting, at this stage, the amount of allyl bromide relative to the amount of sucrose so as to fall within the range of preferably 2-fold to 6-fold moles, and more preferably 2-fold to 5-fold moles. The reaction temperature for etherification can be, for example, about 80°C. Typically, after addition of allyl bromide, the reaction ends in about 3 hours. An alcohol is added to the aqueous phase separated from the reaction mixture, and precipitated salts are filtered, followed by distilling off extra alcohol and water, thereby obtaining sucrose allyl ether.

The polymer containing ethylenically unsaturated carboxylic acid units can be produced by a known method. Examples include a method comprising the step of polymerizing an ethylenically unsaturated carboxylic acid monomer in the presence or absence of a water-soluble crosslinking agent by a suspension polymerization method; and the like. Among suspension polymerization methods, preferable is a reversed-phase suspension polymerization method in which polymerization reaction is performed while droplets of an aqueous phase containing an ethylenically unsaturated carboxylic acid monomer, water, and, if necessary, a water-soluble crosslinking agent are dispersed in a hydrophobic solvent.

When a water-soluble crosslinking agent is used, the proportions of the ethylenically unsaturated carboxylic acid monomer and the water-soluble crosslinking agent are not particularly limited. For example, the amount of the water-soluble crosslinking agent may be 0.01 to 2.0 mass%, relative to the amount of the ethylenically unsaturated carboxylic acid monomer.

Examples of the hydrophobic solvent include petroleum-based hydrocarbon solvents selected from aliphatic hydrocarbons, alicyclic hydrocarbons, and aromatic hydrocarbons. Examples of aliphatic hydrocarbons include n-pentane, n-hexane, n-heptane, and the like. Examples of alicyclic hydrocarbons include cyclopentane, methylcyclopentane, cyclohexane, methylcyclohexane, and the like. Examples of aromatic hydrocarbons include benzene, toluene, xylene, and the like. In particular, at least one hydrophobic solvent selected from n-hexane, n-heptane, cyclohexane, and toluene can suitably be used as an industrially versatile solvent. The amount of the hydrophobic solvent may be, for example, 100 to 200 parts by mass, per 100 parts by mass of the aqueous phase containing the ethylenically unsaturated carboxylic acid monomer etc.

The aqueous phase containing the ethylenically unsaturated carboxylic acid monomer etc. or the hydrophobic solvent may contain other components, such as a surfactant and a radical initiator.

The surfactant is used largely for the purpose of stabilizing the suspension state during polymerization. The surfactant is not particularly limited as long as it is a surfactant typically used in reversed-phase suspension polymerization. Preferable examples of surfactants for use include sorbitan fatty acid esters, polyglycerol fatty acid esters, sucrose fatty acid esters, sorbitol fatty acid esters, modified polyethylene wax, modified polypropylene wax, polyvinyl alcohols, polyethylene oxide, cellulose ethers (e.g., hydroxyethyl cellulose and ethyl cellulose), sodium alkylbenzene sulfonate, polyoxyethylene alkylphenyl ether sulfate, and the like. These surfactants may be used singly, or in a combination of two or more.

The amount of the surfactant is preferably 0.1 to 5.0 mass%, and more preferably 0.2 to 3.0 mass%, relative to the ethylenically unsaturated carboxylic acid monomer.

The radical initiator is not particularly limited as long as it is a radical initiator typically used in radical polymerization. Preferable examples of radical initiators for use include potassium persulfate, ammonium persulfate, sodium persulfate, azo-based initiators, and the like. For example, 2,2'-azobis(2-methylpropionamidine) dihydrochloride can be used as a radical initiator.

The amount of the radical initiator is preferably 0.01 to 0.5 mass%, and more preferably 0.02 to 0.2 mass%, relative to the ethylenically unsaturated carboxylic acid monomer.

In reversed-phase suspension polymerization, the molecular weight and the degree of crosslinkage of the crosslinked polymer can be adjusted by the amount of the water-soluble crosslinking agent added.

Other conditions for polymerization reaction, such as the reaction temperature and reaction time, can also be suitably adjusted. The reaction temperature is, for example, 50 to 80°C, and the reaction time is, for example, 30 minutes to 3 hours. When a 2000-mL flask is used as a reactor, the bath temperature can be adjusted to 60°C to start polymerization reaction. In this case, the start of polymerization reaction can be confirmed by an elevation of the temperature inside the reactor to about 70°C due to heat of polymerization. Thereafter, the polymerization reaction continues for about 30 minutes to 3 hours and then typically ends. After completion of the reaction, the bath temperature is increased to distill off water and the petroleum-based hydrocarbon solvent inside the reactor, thereby obtaining a polymer.

The shape of the component (A) is not particularly limited, and the component (A) is preferably in the form of particles, especially truly spherical or ellipsoidal particles.

After polymerization, crosslinking (post-crosslinking) may be further performed using the water-soluble crosslinking agent described above. Post-crosslinking can be performed when no water-soluble crosslinking agent is used during polymerization or can be further performed after a water-soluble crosslinking agent is used during polymerization. In both cases in which post-crosslinking is not performed and in which post-crosslinking is performed, the proportions of the ethylenically unsaturated carboxylic acid monomer and the water-soluble crosslinking agent are not particularly limited. For example, the amount of the water-soluble crosslinking agent may be 0.01 to 2.0 mass%, relative to the amount of the ethylenically unsaturated carboxylic acid monomer.

The partially neutralized product of a polymer containing ethylenically unsaturated carboxylic acid units can be obtained, for example, by treating (i.e., neutralizing) the polymer with an alkali. Alternatively, the partially neutralized product can be obtained by treating (i.e., neutralizing) the ethylenically unsaturated carboxylic acid monomer beforehand with an alkali and polymerizing the monomer. Preferable examples of alkalis for use include alkali metal salts (e.g., sodium hydroxide and potassium hydroxide), ammonium salts, and the like.

As described above, glycerol (which may be simply referred to as "the component (B)") is present in an amount of 0 to 50 mass%. A content of 0 mass% means that glycerol is not contained. Thus, glycerol may not be contained, and when glycerol is contained, the content of glycerol is 50 mass% or less. The upper or lower limit of the content range (0 to 50 mass%) may be, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49 mass%. The content of glycerol is preferably, for example, 0 to 45 mass%, 5 to 45 mass%, or 10 to 45 mass%.

As described above, the dihydric alcohol (which may be simply referred to as "the component (C)") is present in an amount of 5 to 65 mass%. The upper or lower limit of the content range may be, for example, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, or 64 mass%. The content of the dihydric alcohol is preferably, for example, 10 to 65 mass%, 20 to 60 mass%, or 25 to 60 mass%. Preferable examples of usable dihydric alcohols include propylene glycol, 1,3-butylene glycol, and pentylene glycol. Of these, 1,3-butylene glycol is particularly preferable. The dihydric alcohols may be used singly, or in a combination of two or more.

As described above, water (which may be simply referred to as "the component (D)") is present in an amount of 0 to 20 mass%. A content of 0 mass% means that water is not contained. Thus, water may not be contained, and when water is contained, the content of water is 20 mass% or less. The upper or lower limit of the content range (0 to 20 mass%) may be, for example, 1, 2, 3, 4, 5, 6, 7, 8, 9, 10, 11, 12, 13, 14, 15, 16, 17, 18, or 19 mass%. The content range is preferably, for example, 0 to 15 mass% or 0 to 10 mass%.

As described above, the total content of the components (B) and (C) is 40 mass% or more. The total content of the components (B) and (C) is preferably 40 to 90 mass%. The upper or lower limit of the total content range may be, for example, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, or 89 mass%. The total content of the components (B) and (C) is more preferably, for example, 40 to 85 mass%, 45 to 80 mass%, 50 to 75 mass%, 55 to 70 mass%, or 60 to 70 mass%.

As described above, the mass ratio of the total of the components (B) and (C) to the component (A), i.e., ((B)+(C))/(A), is 1.5 or more. The mass ratio is preferably 1.5 to 3. The upper or lower limit of the mass ratio range may be, for example, 1.6, 1.7, 1.8, 1.9, 2, 2.1, 2.2, 2.3, 2.4, 2.5, 2.6, 2.7, 2.8, or 2.9. The mass ratio range is more preferably, for example, 1.7 to 2.8, 1.8 to 2.5, 1.9 to 2.4, or 2 to 2.3.

The mass ratio of the component (C) to the component (B), i.e., (C)/(B), is preferably 0.5 to 5. The upper or lower limit of the mass ratio range may be, for example, 1, 1.5, 2, 2.5, 3, 3.5, 4, or 4.5. The mass ratio range is more preferably, for example, 0.5 to 4.5, 0.5 to 4, or 1 to 4.

The viscous composition of the present disclosure may contain other additives known in this field (especially in the field of cosmetics), for example, warming-effect-imparting components, such as phenoxyethanol, *Capsicum* extracts, and zeolite; oil components, such as olive oil; plant extracts; and antimicrobial agents, as necessary, as long as effects of the invention are not impaired.

The viscous composition of the present disclosure has a viscosity of preferably 10000 mPa·s or less, more preferably 8000 mPa·s or less, and even more preferably 1000 mPa·s because of ease of handling. The viscosity is measured at an ordinary temperature (25°C) using a Brookfield viscometer (model number: DV1MRVTJ0) at a rotation speed of 20 revolutions per minute. For rotors used in the measurement, rotor No. 3 is used for the viscosity of less than 2000 mPa·s, rotor No.4 is used for the viscosity of 2000 mPa·s or more and less than 5000 mPa·s, rotor No.5 is used for the viscosity of 5000 mPa·s or more and less than 15000 mPa·s, rotor No.6 is used for the viscosity of 15000 mPa·s or more and less than 40000 mPa·s, and rotor No.7 is used for the viscosity of 40000 mPa·s or more and less than 190000 mPa·s.

The pH of the viscous composition of the present disclosure is not particularly limited, and is, for example, preferably about 6.5 to 8, and more preferably about 6.8 to 7.8. The pH is a value measured using a pH meter at 25°C.

The viscous composition of the present disclosure can be easily returned to a uniform viscous composition (for example, by hand stirring) even if layer separation occurs when the composition is stored for a relatively long period of time (e.g., 6 weeks). Thus, even after being stored for a relatively long period of time, the viscous composition of the present disclosure can be used as a uniform viscous composition for the preparation of products such as cosmetics.

The viscous composition of the present disclosure can be prepared, for example, by using the components (A) to (D), optionally with other additives, in predetermined amounts, and mixing them. An example of the method for producing the viscous composition is a method comprising the steps of dispersing the component (A) in the components (B) and (C), adding a predetermined amount of the component (D) thereto to thicken the dispersion, and mixing the remaining components (B) and (C) with the dispersion as necessary, in this order. In particular, the component (A) is excellent in dispersibility in the components (B) and (C), thus making it easy to prepare the viscous composition.

In this specification, the terms "comprising" and "containing" include "consisting essentially of" and "consisting of." Further, the present disclosure includes all of any combinations of the constituent requirements described in the present specification.

In addition, the various characteristics (properties, structures, functions, etc.) described in each embodiment of the present disclosure described above may be combined in any way in specifying the subjects included in the present disclosure. In other words, the present disclosure includes all the subjects comprising all combinations of the combinable characteristics described in the present specification.

### Examples

The subjects of the present disclosure are described in more detail below; however, the subjects of the present disclosure are not limited to the following examples.

### Synthesis of Water-soluble Crosslinking Agent

A stirrer, a reflux condenser, and a dropping funnel were attached to a 1000-mL separable flask. In the flask, 48 g of sodium hydroxide was dissolved in 144 g of water. Subsequently, 136.8 g of sucrose was added thereto, and the mixture was stirred at 70 to 85°C for 120 minutes, thereby obtaining an aqueous solution of alkaline sucrose. 145.2g of allyl bromide was added dropwise to the obtained aqueous solution of alkaline sucrose at 70 to 85°C over the course of 1.5 hours, and then the mixture was reacted at 80°C for 3 hours to allyl-etherify the sucrose. After cooling, 440 g of water was added thereto, and excessive oil was removed with a separatory funnel, thereby obtaining an aqueous solution of crude sucrose allyl ether. Hydrochloric acid was added to the obtained crude sucrose allyl ether to adjust the pH to 6 to 8, and water was removed with a rotary evaporator until the mass of the aqueous solution reached 480 g. Subsequently, 200 g of ethanol was added thereto, and salts, such as sodium bromide, (by-products) were precipitated, followed by removing the precipitates from the aqueous solution through filtration. Further, excessive water was removed from the aqueous solution with an evaporator, thereby obtaining 166 g of sucrose allyl ether with a degree of etherification of 2.4.

### Synthesis of Partially Neutralized Product of Polymer Containing Ethylenically Unsaturated Carboxylic Acid Units

### Production Example 1

A stirrer, a reflux condenser, and a dropping funnel were attached to a 500-mL separable flask. To the flask, 72 g of acrylic acid and water were added to prepare 90 g of an 80 mass% aqueous acrylic acid solution. While this aqueous acrylic acid solution was cooled, 54 g of a 30 mass% aqueous sodium hydroxide solution was added dropwise thereto to prepare an aqueous acrylic acid neutralized solution with a degree of neutralization of 40%. Subsequently, 0.32 g of the sucrose allyl ether obtained above and 0.04 g of 2,2'-azobis(2-methylpropionamidine) dihydrochloride (V-50 produced by Wako Pure Chemical Industries, Ltd.,) were added thereto, thereby preparing a starting material aqueous solution for synthesis.

Separately, 330 g of n-heptane was added to a 2000-mL separable flask equipped with a stirrer, a reflux condenser, a dropping funnel, and a nitrogen gas inlet tube, and 2.7 g of sorbitan monostearate (NONION SP-60R produced by NOF Corporation) was further added thereto, followed by dispersing and dissolving it in n-heptane. Subsequently, the starting material aqueous solution for synthesis prepared in advance was added thereto. To remove the oxygen present in the atmosphere in the reactor, in the starting materials, and in the solvent, nitrogen gas was blown into the solution. While the inside of the system was replaced with nitrogen, a reaction was performed for 1 hour with the bath temperature maintained at 60°C, with stirring at a rotational frequency of 1000 rotations/minute. After completion of the reaction, water and n-heptane were distilled off, thereby obtaining a polymer of acrylic acid and a sodium salt thereof (a sodium salt of a carboxyvinyl polymer; degree of neutralization: 40 mol%). This polymer may be referred to below as "Production Example 1 polymer."

### Production Example 2

A 1000-ml five-necked cylindrical round-bottom flask equipped with a reflux condenser, a dropping funnel, a nitrogen gas inlet tube, a stirrer, and a stirring blade was prepared. In the flask, 340 g of n-heptane was placed, and 0.92 g of a sucrose stearic acid ester having an HLB of 3 (Ryoto Sugar Ester S-370 produced by Mitsubishi-Kagaku Foods Corporation) and 0.92 g of maleic anhydride modified ethylene propylene copolymer (Hi-Wax 1105A produced by Mitsui Chemicals, Inc.) were added thereto. The mixture was heated to 80°C with stirring to dissolve the surfactant, and then cooled to 55°C.

92 g (1.02 mol) of an 80 mass% aqueous acrylic acid solution was placed in a 500-ml Erlenmeyer flask. While the flask was cooled from the outside, 54.5 g (0.41 mol) of a 30 mass% aqueous sodium hydroxide solution was added dropwise thereto to perform 40 mol% neutralization. 1.15 g of a 2.0 mass% aqueous 2,2'-azobis(2-amidinopropane)dihydrochloride solution as a radical polymerization initiator, 0.92 g of a 1.0 mass% aqueous sodium hypophosphite monohydrate solution, and 51.6 g of ion-exchanged water were added thereto and dissolved, thereby preparing a monomer aqueous solution.

The entire quantity of the monomer aqueous solution was added to the cylindrical round-bottom flask. The flask was dipped in a 60°C water bath to heat it to 58°C. The inside of the system was replaced with nitrogen, followed by conducting a polymerization reaction. The contents reached the peak temperature (79°C) 30 minutes after the initiation of the polymerization reaction. The flask was maintained in the state of being dipped in the 60°C water bath for 30 minutes, and the reaction was continued. The temperature of the internal solution after 30 minutes was 59°C. The resulting polymerization slurry liquid was cooled to 30°C, thereby obtaining a polymer (a sodium salt of a carboxyvinyl polymer; degree of neutralization: 40 mol%). This polymer may be referred to below as "Production Example 2 polymer."

### Production Example 3

92.0 g of an 80 mass% aqueous acrylic acid solution was placed in a 500-mL Erlenmeyer flask. While the flask was cooled from the outside, 102.2 g of 30 mass% sodium hydroxide was added dropwise thereto with stirring to perform neutralization. 0.073 g of potassium persulfate, 10.1 mg of ethylene glycol diglycidyl ether, and 44.0 g of distilled water were added thereto, thereby preparing a monomer aqueous solution.

As a petroleum-based hydrocarbon dispersion medium, 282 g of normal heptane (referred to below as "dispersion medium" in the description of Production Example 3) was weighed in a 2-L five-necked round-bottom flask equipped with a stirrer with one set of 50-mm-diameter slanted paddle blades, a thermometer, a reflux condenser, and a nitrogen gas inlet tube. To the round-bottom flask, 0.74 g of a sucrose fatty acid ester (trade name: S-370, produced by Mitsubishi-Kagaku Foods Corporation) as a surfactant and 0.74 g of maleic anhydride modified ethylene propylene copolymer (trade name: Hi-Wax 1105A, produced by Mitsui Chemicals, Inc.) as a hydrophobic polymer dispersing agent were added. The mixture was heated to about 80°C in a hot-water bath at 85°C with stirring at 300 rpm for dissolution and dispersion, and then air-cooled to an internal temperature of 64°C. The monomer aqueous solution was added to the dispersion medium stirred at a speed increased to 500 rpm, at one time, using an SUS funnel with an opening having an inner diameter of 8 mm at the tip. After addition of the monomer aqueous solution, the inside of the system was sufficiently replaced with nitrogen while the internal temperature was maintained at 40°C. Thereafter, the mixture was heated for 1 hour using a hot-water bath at 70°C to perform a radical polymerization reaction.

After the polymerization reaction, the stirring speed was increased to 1000 rpm, and 100 g of dispersion medium was added to the flask. The mixture was heated using an oil bath at 120°C, and 125 g of water was removed from the system by azeotropic distillation while the dispersion medium was refluxed in the flask, thereby obtaining a dehydrated polymer dispersed in the dispersion medium. To the obtained dehydrated polymer in the dispersion medium, 3.4 g of a 2% aqueous ethylene glycol diglycidyl ether solution was added as a post-crosslinking agent, and a post-crosslinking reaction was performed at 83°C for 2 hours.

Thereafter, heating was performed using an oil bath at 120°C, and the dispersion medium and water was removed from the system by distillation, followed by drying under nitrogen flow and passing through a 850 µm-sieve, thereby obtaining 91 g of a resin (sodium salt of a carboxyvinyl polymer; degree of neutralization: 75 mol%). This resin may be referred below to as "Production Example 3 polymer."

### Synthesis of Carboxyvinyl Polymer

### Production Example 4

45 g (0.625 mol) of acrylic acid, 0.27 g of pentaerythritol tetraallyl ether, 150 g of n-hexane, and 0.081 g (0.00035 mol) of 2,2'-azobis methyl isobutyrate were placed in a 500-mL four-necked flask equipped with a stirrer, a thermometer, a nitrogen blowing tube, and a condenser, thereby preparing a reaction solution. The solution was stirred and mixed uniformly, and then nitrogen gas was blown into the solution to remove oxygen present in the upper space of the reactor, in the starting materials, and in the solvent. Subsequently, the reaction solution was reacted for 4 hours in a nitrogen atmosphere with the temperature of the reaction solution maintained at 60 to 65°C. After completion of the reaction, the generated slurry was heated to 90°C to distill off n-hexane, further followed by drying under reduced pressure at 110°C at 10 mmHg for 8 hours, thereby obtaining 42 g of a carboxyvinyl polymer. This polymer may be referred to below as "Production Example 4 polymer."

### Preparation and Examination of Viscous Composition

Viscous compositions of Examples and Comparative Examples were prepared using the obtained polymers of Production Examples 1 to 4, according to the formulations shown in Table 1. Specifically, glycerol and 1,3-butylene glycol were mixed at 400 rpm with a three-one motor (BL1200, general-purpose stirrer, produced by Shinto Scientific Co., Ltd.) for 1 minute. To the mixture of glycerol and 1,3-butylene glycol, each of the polymers of Production Examples 1 to 4 was individually gradually added in two or three divided portions with stirring using the three-one motor. Since the viscosity increases with the addition of each polymer, the mixture was stirred for 8 minutes while the stirring speed was gradually increased to 1000 rpm until the mixture became uniform. In each of the examples in which water was added, ion-exchanged water was added at one time with stirring at 1000 rpm, and the mixture was further stirred for 8 minutes, thereby obtaining a uniform mixture. The viscous compositions were prepared by mixing the components in this manner.

The numerical values of the components listed in Table 1 are expressed as percentages based on the total amount taken as 100%. The actual preparation was performed on a scale of 50 g in total.

The viscosity and pH of the obtained viscous compositions were measured. Moreover, the viscous compositions were allowed to stand for 6 weeks after preparation, and then the state of the viscous compositions was observed and examined. Table 1 also shows the results. ND indicates that examination was not performed.

The viscosity was measured at an ordinary temperature (25°C) using a Brookfield viscometer (model number: DV1MRVTJ0) at a rotation speed of 20 revolutions per minute. For rotors used in the measurement, rotor No. 3 was used for the viscosity of less than 2000 mPa·s, rotor No. 4 was used for the viscosity of 2000 mPa·s or more and less than 5000 mPa·s, rotor No. 5 was used for the viscosity of 5000 mPa·s or more and less than 15000 mPa·s, rotor No. 6 was used for the viscosity of 15000 mPa·s or more and less than 40000 mPa·s, and rotor No. 7 was used for the viscosity of 40000 mPa·s or more and less than 190000 mPa·s. The pH was measured with a pH meter at 25°C.

Further, 1 g of each of the prepared viscous compositions was individually weighed and mixed with 99 g of water. All of the viscous compositions of the Examples were dispersed well (uniformly) in water, whereas each of the viscous compositions of the Comparative Examples was not dispersed uniformly.

The above results show that all of the viscous compositions of the Examples are easily returned to viscous compositions with the same level of uniformity as those at the time of preparation, by hand stirring, even after they are allowed to stand for 6 weeks after preparation.

**Table 1**

| | Production Example 1 polymer | Production Example 2 polymer | Production Example 3 polymer | Productio n Example 4 polymer | Glycerol | 1,3-Butylene glycol | Ion-exchanged water | Polysorbate 60 | Diethylenetriaminepentaacetic acid | Viscosity at the time of preparation (mPa·s) | State 6 weeks after preparation | pH |
|---|---|---|---|---|---|---|---|---|---|---|---|---|
| Ex. 1 | 30.0 | - | - | - | 14.0 | 56.0 | - | - | - | 520 | Separated into two layers, became uniform by hand stirring with spatula | 7.8 |
| Ex. 2 | 30.0 | - | - | - | 28.0 | 42.0 | - | - | - | 1,060 | Separated into two layers, became uniform by hand stirring with spatula | 7.5 |
| Ex 3 | 30.0 | - | - | - | 35.0 | 35.0 | - | - | - | 1,280 | Separated into two layers, became uniform by hand stirring with spatula | 7.6 |
| Ex.4 | 30.0 | - | - | - | 42.0 | 28.0 | - | - | - | 6,140 | Separated into three layers, became uniform by hand stirring with spatula | 7.5 |
| Ex. 5 | 30.0 | - | - | - | 13.0 | 52.0 | 5.0 | - | - | 260 | Separated into two layers, became uniform by hand stirring with spatula | 7.1 |
| Ex 6 | 30.0 | - | - | - | 12.0 | 48.0 | 10.0 | - | - | 200 or less | Separated into two layers, became uniform by hand stirring with spatula | 7.0 |
| Ex.7 | 30.0 | - | - | - | - | 60.0 | 10.0 | - | - | 320 | Separated into two layers, became uniform by hand stirring with spatula | ND |
| Ex 8 | - | 30.0 | - | - | 14.0 | 56.0 | - | - | - | 340 | Separated into two layers, became uniform by hand stirring with spatula | 6.9 |
| Ex.9 | - | - | 30.0 | - | 14.0 | 56.0 | - | - | - | 380 | Separated into two layers, became uniform by hand stirring with spatula | 7.3 |
| Comp . Ex 1 | 50.0 | - | - | - | - | 50.0 | - | - | - | Non-uniform | Solidification of lower layer | ND |
| Comp . Ex. 2 | 60.0 | - | - | - | - | 40.0 | - | - | - | 8750 (non-uniform) | Solidification of lower layer | 6.0 |
| Comp . Ex3 | 60.0 | - | - | - | - | 39.28 | - | - | 0.2952 | 9400 (non-uniform) | ND | 6.3 |
| Comp . Ex. 4 | 70.0 | - | - | - | - | 30.0 | - | - | - | Non-uniform | ND | ND |
| Comp . Ex. 5 | 50.0 | - | - | - | - | 48.6 | - | - | 0.0056 | Non-uniform | ND | ND |
| Comp .Ex.6 | 30.0 | - | - | - | 10.0 | 40.0 | 30.0 | - | - | Non-uniform | ND | ND |
| Comp .Ex.7 | 60.0 | - | - | 1.0 | - | 39.0 | - | - | - | Non-uniform | ND | ND |
| Comp . Ex.8 | 40.0 | - | - | 4.0 | - | 56.0 | - | - | - | 181,000 | ND | ND |
| Comp .Ex.9 | 30.0 | - | - | - | 56.0 | 14.0 | - | - | - | 4,950 | Solidification of glycerol layer (lower layer) | 7.1 |
| Comp . Ex 10 | 30.0 | - | - | - | - | 40.0 | 30.0 | - | - | 2,520 | Non-uniform gelation | ND |
| Comp . Ex. 11 | 30.0 | - | - | - | - | 70.0 | - | - | - | uniform | Solidification of lower layer | ND |
| Comp . Ex. 12 | 30.0 | - | - | - | - | 68.0 | - | 2.0 | - | 280 or less | Solidification of lower layer | ND |
| Comp . Ex. 13 | 39.6 | - | - | 1.8 | - | 58.7 | - | - | - | 5,100 | Crystallization of lower layer | 5.6 |
| Comp . Ex. 14 | 50.0 | - | - | 1.8 | - | 48.3 | - | - | - | 26,800 | Crystallization of lower layer | 5.8 |
| Comp . Ex. 15 | - | - | - | 30.0 | 14.0 | 56.0 | - | - | - | Could not be prepared because of overly high viscosity | ND | ND |

## Claims

1. A viscous composition comprising:
(A) 10 to 40 mass% of a partially neutralized product of a polymer containing ethylenically unsaturated carboxylic acid units;
(B) 0 to 50 mass% of glycerol;
(C) 5 to 65 mass% of a dihydric alcohol; and
(D) 0 to 20 mass% of water,
the total of the components (B) and (C) being 40 mass% or more,
the mass ratio of the total of the components (B) and (C) to the component (A), ((B)+(C))/(A), being 1.5 or more.

2. The viscous composition according to claim 1, wherein the mass ratio of the component (C) to the component (B), (C)/(B), is 0.5 to 5.

3. The viscous composition according to claim 1 or 2, wherein the partially neutralized product of a polymer containing ethylenically unsaturated carboxylic acid units is crosslinked or is not crosslinked by a water-soluble crosslinking agent.

4. The viscous composition according to claim 1 or 2, wherein the partially neutralized product of a polymer containing ethylenically unsaturated carboxylic acid units is crosslinked by a water-soluble crosslinking agent, and the water-soluble crosslinking agent is at least one member selected from the group consisting of ethylene glycol diglycidyl ether, pentaerythritol allyl ether, and sucrose allyl ether.

5. The viscous composition according to any one of claims 1 to 4, wherein the dihydric alcohol (C) is present in an amount of 20 to 65 mass%.

6. The viscous composition according to any one of claims 1 to 5, wherein the mass ratio of the total of the components (B) and (C) to the component (A), ((B)+(C))/(A), is 1.5 to 3.

7. The viscous composition according to any one of claims 1 to 6, wherein the partially neutralized product of a polymer containing ethylenically unsaturated carboxylic acid units is a partially neutralized product of a (meth)acrylic acid polymer.

8. The viscous composition according to any one of claims 1 to 7, wherein the partially neutralized product of a polymer containing ethylenically unsaturated carboxylic acid units is a neutralized product in which 30 to 80 mol% of all the ethylenically unsaturated carboxylic acid units contained in the polymer are neutralized.

9. The viscous composition according to any one of claims 1 to 8, wherein the dihydric alcohol (C) is at least one member selected from the group consisting of propylene glycol, 1,3-butylene glycol, and pentylene glycol.

10. The viscous composition according to claim 1, wherein the partially neutralized product of a polymer containing ethylenically unsaturated carboxylic acid units (A) is (i) a partially neutralized product of a (meth)acrylic acid polymer that is not crosslinked by a water-soluble crosslinking agent or (ii) a partially neutralized product of a (meth)acrylic acid polymer that is crosslinked by at least one water-soluble crosslinking agent selected from the group consisting of ethylene glycol diglycidyl ether, pentaerythritol allyl ether, and sucrose allyl ether;
the partially neutralized product is a neutralized product in which 30 to 80 mol% of (meth)acrylic acid units contained in the (meth)acrylic acid polymer are neutralized;
the dihydric alcohol (C) is at least one dihydric alcohol selected from the group consisting of propylene glycol, 1,3-butylene glycol, and pentylene glycol, and is present in an amount of 20 to 65 mass%;
the mass ratio of the component (C) to the component (B), (C)/(B), is 0.5 to 5; and
the mass ratio of the total of the components (B) and (C) to the component (A), ((B)+(C))/(A), is 1.5 to 3.
